# EUROPEAN PATENT SPECIFICATION

(11) **EP 4 719 203 B1**
(45) Date of publication and mention of the grant of the patent: **08.07.2026**
(21) Application number: 24729737.7
(22) Date of filing: 23.05.2024
(51) Int. Cl.: A61B 8/08, A61B 8/00, G06T 7/00, G06T 7/11, G16H 50/30

(54) **SYSTEM FOR DIAGNOSING SARCOPENIA**
SYSTEM ZUR DIAGNOSE VON SARKOPENIE
SYSTÈME DE DIAGNOSTIC DE LA SARCOPÉNIE

(30) Priority: 26.05.2023 WO PCT/CN2023/096500
(43) Date of publication of application: 08.04.2026
(73) Proprietor: Koninklijke Philips N.V., 5656 AE Eindhoven (NL)
(72) Inventor: XU, Jingping, 5656 AG Eindhoven (NL); DENG, Junping, 5656 AG Eindhoven (NL); MENG, Yishuang, 5656 AG Eindhoven (NL)
(74) Representative: Philips Intellectual Property & Standards
(86) International application number: PCT/EP2024/064159
(87) International publication number: WO 2024/245868

(56) References cited:
- KR-A- 20200 120 076
- KR-A- 20220 095 803
- KR-B1- 102 405 320
- PARK B. ET AL: "Predicting intramuscular fat in beef longissimus muscle from speed of sound", JOURNAL OF ANIMAL SCIENCE, vol. 72, no. 1, 1 January 1994 (1994-01-01), United States, pages 109 - 116, XP093189484, ISSN: 0021-8812, Retrieved from the Internet <URL:https://dx.doi.org/10.2527/1994.721109x> DOI: 10.2527/1994.721109x
- MANZANO WILFRED ET AL: "Sarcopenia in rheumatic disorders: what the radiologist and rheumatologist should know", SKELETAL RADIOLOGY, SPRINGER, BERLIN, DE, vol. 51, no. 3, 16 July 2021 (2021-07-16), pages 513 - 524, XP037668188, ISSN: 0364-2348, [retrieved on 20210716], DOI: 10.1007/S00256-021-03863-Z
- RUBY LISA ET AL: "Speed of sound ultrasound: comparison with proton density fat fraction assessed with Dixon MRI for fat content quantification of the lower extremity", EUROPEAN RADIOLOGY, SPRINGER BERLIN HEIDELBERG, BERLIN/HEIDELBERG, vol. 30, no. 10, 8 May 2020 (2020-05-08), pages 5272 - 5280, XP037238865, ISSN: 0938-7994, [retrieved on 20200508], DOI: 10.1007/S00330-020-06885-8
- SANABRIA SERGIO J ET AL: "Speed of sound ultrasound: a pilot study on a novel technique to identify sarcopenia in seniors", EUROPEAN RADIOLOGY, SPRINGER BERLIN HEIDELBERG, BERLIN/HEIDELBERG, vol. 29, no. 1, 15 October 2018 (2018-10-15), pages 3 - 12, XP036657799, ISSN: 0938-7994, [retrieved on 20181015], DOI: 10.1007/S00330-018-5742-2
- PRASAD S ET AL: "DeepUCT: Complex cascaded deep learning network for improved ultrasound tomography", 20220311, vol. 67, no. 6, 11 March 2022 (2022-03-11), XP020457440, DOI: 10.1088/1361-6560/AC5296
- KORTA MARTIARTU NAIARA ET AL: "Toward Speed-of-Sound Anisotropy Quantification in Muscle With Pulse-Echo Ultrasound", IEEE TRANSACTIONS ON ULTRASONICS, FERROELECTRICS, AND FREQUENCY CONTROL, IEEE, USA, vol. 69, no. 8, 6 July 2022 (2022-07-06), pages 2499 - 2511, XP011915764, ISSN: 0885-3010, [retrieved on 20220707], DOI: 10.1109/TUFFC.2022.3189184

## Description

### BACKGROUND

Sarcopenia is a chronic disease characterized by a progressive loss in the amount, composition, and function of skeletal muscle in the human body. Generally, for a patient with sarcopenia, muscular fibers are replaced by intramuscular adipose tissue (fat content). The prevalence of sarcopenia varies between 10 percent and 27 percent globally, with severe sarcopenia varying between 2 percent and 9 percent globally. Older age may be the most significant risk factor, although additional reported risk factors independently associated with sarcopenia include household status, lifestyle, physical inactivity, poor nutritional and dental status, and disease (e.g., osteoporosis, metabolic disease). In particular, the likelihood of developing sarcopenia is correlated with a number of cardio-metabolic risk factors, notably including diabetes, hypertension, dyslipidemia, loss of motor neurons, less active neuromuscular junction, hormonal status, pro-inflammatory cytokines, decreased mitochondrial function, abnormal myokine production, and weight loss accompanying decreased appetite. Screening and early diagnosis of sarcopenia already represent major medical and social challenges due to the generally aging society worldwide. Notably, ultrasound imaging is emerging as an important tool for measuring muscle quantity and quality. Also, ultrasound imaging provides the unique benefit of being a non-invasive modality that is usable at bedside for making serial measurements.

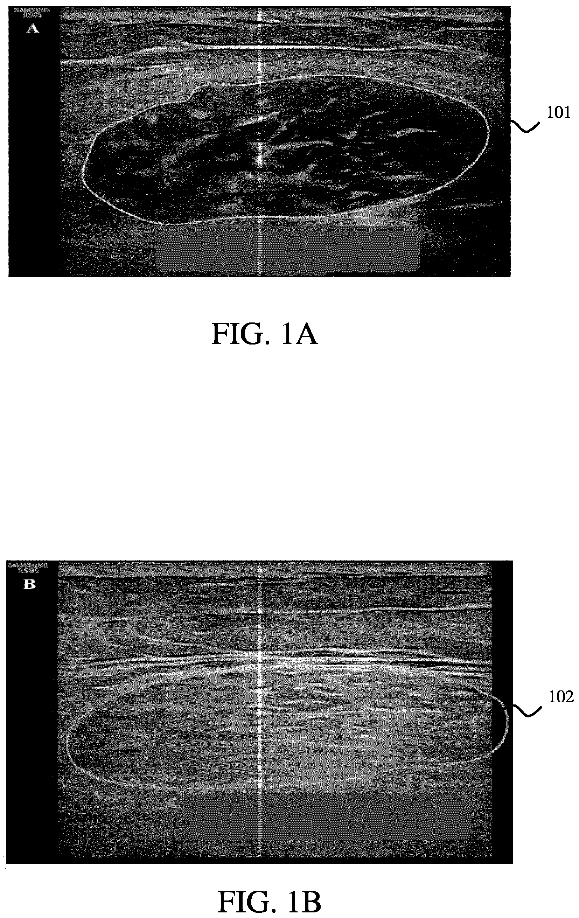

Ultrasound has been used for muscle function evaluation, including texture analysis and speed of sound (SoS) ultrasound. For example, FIGs. 1A and 1B show transverse ultrasound images of the proximal third of the rectus femoris of two female patients with similar age and body mass index (BMI). In particular, image 101 in FIG. 1A shows the rectus femoris of a 38-year-old first patient with a BMI of 24.2 who is an avid runner, while image 102 in FIG. 1B shows the rectus femoris of a 42-year-old second patient with a BMI of 25.1 who does not practice any physical activity. Both patients have similar muscle trophisms, in that the cross-sectional area (CSA) index of the first patient is 7.575 cm² and the CSA of the second patient is 7.351 cm². Regardless, an increased muscle belly echogenicity can be observed in the second patient due to fatty infiltration detectable in image 102.

However, conventional workflows for sarcopenia evaluation using ultrasound imaging is based on simple parameters, such as muscle thickness and echogenicity, as well as CSA index with several critical methodological issues, which provide conflicting results from previous studies. For example, conventional workflows for muscle ultrasound examinations are complicated and time-consuming, with no advanced tools to help reduce workload. Also, clear standardization is absent in clinical practice.

KR20200120076A discloses an apparatus for predicting sarcopenia using machine learning.

SANABRIA SERGIO J ET AL: "Speed of sound ultrasound: a pilot study on a novel technique to identify sarcopenia in seniors", EUROPEAN RADIOLOGY, SPRINGER BERLIN HEIDELBERG, BERLIN/HEIDELBERG, vol. 29, no. 1, 15 October 2018, pages 3-12 ISSN: 0938-7994, DOI: 10.1007/ S00330-018-5742-2 discloses to assess calf muscles of young and elderly females measuring speed of sound, quantifying muscle loss and fatty muscular degeneration.

PRASAD S ET AL: "DeepUCT: Complex cascaded deep learning network for improved ultrasound tomography",20220311, vol. 67, no. 6, 11 March 2022, DOI: 10.1088/1361-6560/AC5296 discloses using ultrasound computed tomography for the quantitative assessment of speed of sound and attenuation.

KORTA MARTIARTU NAIARA ET AL: "Toward Speed-of-Sound Anisotropy Quantification in Muscle With Pulse-Echo Ultrasound",IEEE TRANSACTIONS ON ULTRASONICS, FERROELECTRICS, AND FREQUENCY CONTROL, IEEE, USA, vol. 69, no. 8, 6 July 2022, pages 2499-2511,ISSN: 0885-3010, DOI: 10.1109/TUFFC.2022.3189184 discloses speed of sound as a biomarker of musculoskeletal disorders.

### SUMMARY OF THE DISCLOSURE

The invention is set out in the appended set of claims.

In another representative embodiment, a system is provided for diagnosing sarcopenia in a patient. The system includes a user interface; a processor in communication with the user interface; a non-transitory memory storing instructions that, when executed by the processor, cause the processor to receive a longitudinal ultrasound image acquired by a longitudinal scan of a muscle of interest in the patient using ultrasound imaging; receive a transverse ultrasound image acquired by a transverse scan of the muscle of interest in the patient using ultrasound imaging; segment the longitudinal and transverse ultrasound images to identify targeted muscle regions of the muscle of interest in the longitudinal and the transverse ultrasound images, respectively; determine global attenuations in the target muscle regions between boundaries in the longitudinal and transverse ultrasound images, respectively; identify regions of interest (ROIs) in the targeted muscle regions in the longitudinal and the transverse ultrasound images, respectively; determine local attenuation coefficients of the ROIs in the targeted muscle regions, respectively; determine global speeds of sound (SoSs) in the targeted muscle regions between the boundaries in the longitudinal and transverse ultrasound images, respectively; determine local SoSs in the ROIs in the targeted muscle regions, respectively; and determine a level of sarcopenia in the patient based on the global attenuations and the global SoSs in the targeted muscle regions, and the local attenuation coefficients and the local SOSs in the ROIs. The system further includes a display configured to display an indication of the level of sarcopenia.

In another representative embodiment, a non-transitory computer readable medium storing instructions for diagnosing sarcopenia in a patient that, when executed by a processor, cause the processor to receive a longitudinal ultrasound image acquired by a longitudinal scan of a muscle of interest in the patient using ultrasound imaging; receive a transverse ultrasound image acquired by a transverse scan of the muscle of interest in the patient using ultrasound imaging; segment the longitudinal and transverse ultrasound images to identify targeted muscle regions of the muscle of interest in the longitudinal and the transverse ultrasound images, respectively; determine global attenuations in the target muscle regions between boundaries in the longitudinal and transverse ultrasound images, respectively; identify regions of interest (ROIs) in the targeted muscle regions in the longitudinal and the transverse ultrasound images, respectively; determine local attenuation coefficients of the ROIs in the targeted muscle regions, respectively; determine global speeds of sound (SoSs) in the targeted muscle regions between the boundaries in the longitudinal and transverse ultrasound images, respectively; determine local SoSs in the ROIs in the targeted muscle regions, respectively; determine a level of sarcopenia in the patient based on the global attenuations and the global SoSs in the targeted muscle regions, and the local attenuation coefficients and the local SOSs in the ROIs; and display an indication of the level of sarcopenia.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1A shows a transverse ultrasound image of the rectus femoris of a first patient who regularly exercises, and has low fatty infiltration of muscle.
FIG. 1B shows a transverse ultrasound image of a second patient who does not exercise, and has high fatty infiltration of muscle.
FIG. 2 is a simplified block diagram of a system for performing a sarcopenia evaluation using ultrasound imaging, according to a representative embodiment.
FIG. 3A shows excellent muscle segmentation results by a deep learning-based segmentation algorithm, according to a representative embodiment.
FIG. 3B shows moderate to poor muscle segmentation results by the deep learning-based segmentation algorithm, according to a representative embodiment.
FIG. 4 is a flow diagram showing a method of evaluating sarcopenia in a subject using ultrasound imaging.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the various embodiments described herein provide a system for implementing an improved workflow for sarcopenia evaluation using ultrasound imaging. The workflow includes automatically segmenting the ROI of muscle under investigation from the ultrasound image; measuring global attenuation for a whole piece of the muscle from the ROI for both longitudinal and transverse scans; measuring local attenuation within a small ROI for a piece of muscle, as well as both longitudinal and transverse scans; measuring SoS in both the longitudinal and transverse scans; and evaluating grading of sarcopenia from the combination of the measured ultrasound parameters.

The workflow reduces the time and effort needed for performing a musculoskeletal (MSK) examination since data acquisition is the same as that required for conventional ultrasound examinations in which muscle clearly appears in the longitudinal and transverse scans. The workflow also simplifies the muscle quality evaluation, especially for relatively inexperienced users (e.g., physicians and sonographers), thereby improving results and increasing the user's confidence. The workflow enables early diagnosis of patients with high risk of sarcopenia by using quantitative muscle ultrasound to reduce effort and examination time.

For patients with sarcopenia, muscular fibers are replaced by intramuscular adipose tissue (fat content), which results in reduction of SoS. This is due to the small SoS in the adipose tissue (1440 m/s) as compared to SoS in muscle (1585 m/s). Correspondingly, attenuation of ultrasound during ultrasound imaging is also reduced along with the intramuscular fat fraction. Since muscle tissue is anisotropic, meaning that certain physical properties have different values when measured in different directions, the ultrasound parameters from both the longitudinal and transverse scans are integrated into the workflow for sarcopenia evaluation, according to the embodiments herein.

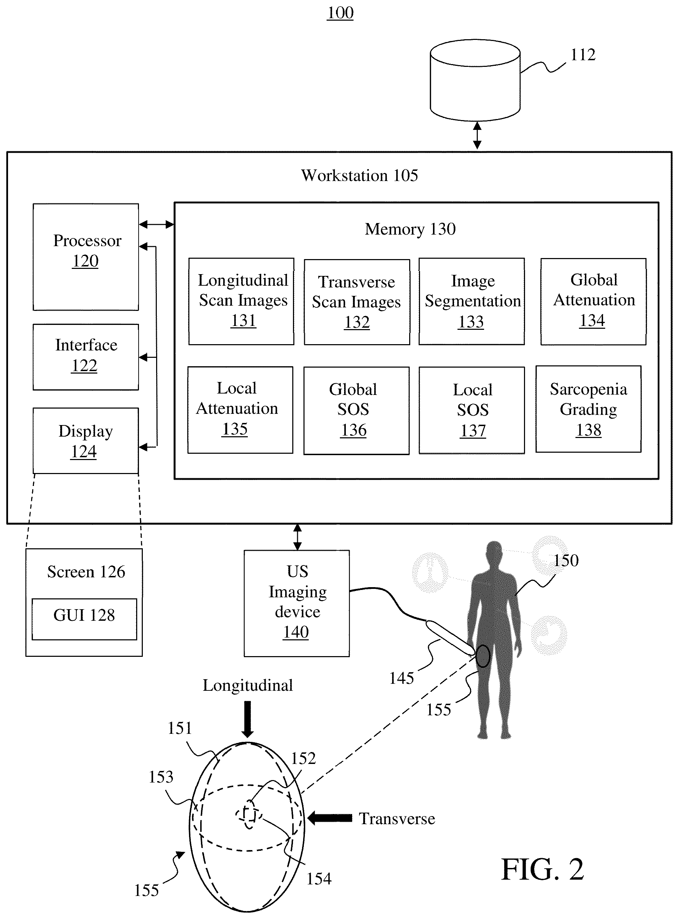

FIG. 2 is a simplified block diagram of a system for performing a sarcopenia evaluation using ultrasound imaging, according to a representative embodiment.

Referring to FIG. 2, system 100 includes a workstation 105 for implementing and/or managing the processes described herein with regard to evaluating sarcopenia of a subject (patient) 150 using ultrasound images from an ultrasound imaging device 140. The workstation 105 includes one or more processors indicated by processor 120, one or more memories indicated by memory 130, a user interface 122 and a display 124. The processor 120 communicates with the ultrasound imaging device 140 through an imaging interface (not shown). The ultrasound imaging device 140 includes an ultrasound transducer probe 145 operable by a user to obtain musculoskeletal (MSK) ultrasound images of a portion of the subject 150. The ultrasound transducer probe 145 may be manipulated manually by the user, automatically by a robot under control of a robot controller (not shown), or a combination of both.

The memory 130 stores instructions executable by the processor 120. When executed, the instructions cause the processor 120 to implement one or more processes for performing a sarcopenia evaluation of the subject 150 using ultrasound images acquired by the ultrasound imaging device 140. The ultrasound images may be provided from the ultrasound imaging device 140 in real-time or near real-time during the scanning procedure, or may be retrieved from storage following the scanning procedure. For purposes of illustration, the memory 130 is shown to include software modules, each of which includes the instructions, executable by the processor 120, corresponding to an associated capability of the system 100.

The processor 120 is representative of one or more processing devices, and may be implemented by a general purpose computer, a central processing unit (CPU), a digital signal processor (DSP), a graphical processing unit, a computer processor, a microprocessor, a state machine, programmable logic device, field programmable gate arrays (FPGAs), application specific integrated circuits (ASICs), or combinations thereof, using any combination of hardware, software, firmware, hard-wired logic circuits, or combinations thereof. Any processor or processing unit herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 130 may include main memory and/or static memory, where such memories may communicate with each other and the processor 120 via one or more buses. The memory 130 may be implemented by any number, type and combination of random access memory (RAM) and read-only memory (ROM), for example, and may store various types of information, such as software algorithms, artificial intelligence (AI) machine learning models, and computer programs, all of which are executable by the processor 120. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

The system 100 may also include a database 112 for storing information that may be used by the various software modules of the memory 130. For example, the database 112 may include image data from previously obtained ultrasound images of the subject 150 and/or of other similarly situated subjects. The stored image data may be used for training an AI machine learning model, such as a neural network model, for example, as discussed below. The database 112 may be implemented by any number, type and combination of RAM and ROM, for example. The various types of ROM and RAM may include any number, type and combination of computer readable storage media, such as a disk drive, flash memory, EPROM, EEPROM, registers, a hard disk, a removable disk, tape, CD-ROM, DVD, floppy disk, Blu-ray disk, USB drive, or any other form of storage medium known in the art. The database 112 comprises tangible storage mediums for storing data and executable software instructions and is non-transitory during the time data and software instructions are stored therein. The database 112 may be secure and/or encrypted, or unsecure and/or unencrypted. For purposes of illustration, the database 112 is shown as a separate storage medium, although it is understood that it may be combined with and/or included in the memory 130, without departing from the scope of the present teachings.

The processor 120 may include or have access to an artificial intelligence (AI) engine, which may be implemented as software that provides artificial intelligence (e.g., neutral network models) and applies machine learning described herein. The AI engine may reside in any of various components in addition to or other than the processor 120, such as the memory 130, an external server, and/or the cloud, for example. When the AI engine is implemented in a cloud, such as at a data center, for example, the AI engine may be connected to the processor 120 via the internet or other communication network using one or more wired and/or wireless connection(s).

The user interface 122 is configured to provide information and data output by the processor 120, the memory 130 and/or the ultrasound imaging device 140 to the user and/or for receiving information and data input by the user. That is, the user interface 122 enables the user to enter data and to control or manipulate aspects of the processes described herein, and also enables the processor 120 to indicate the effects of the user's input, which may include control or manipulation of the ultrasound transducer probe 145. All or a portion of the user interface 122 may be implemented by a graphical user interface (GUI), such as GUI 128 viewable on the display 124, discussed below. The user interface 122 may include one or more interface devices, such as a mouse, a keyboard, a trackball, a joystick, a microphone, a video camera, a touchpad, a touchscreen, voice or gesture recognition captured by a microphone or video camera, for example.

The display 124 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 124 includes a screen 126 for viewing ultrasound images of the subject 150, along with various features described herein to communicate to the user the degree of sarcopenia, if any, indicated in the images, as well as the GUI 128 to enable the user to interact with the displayed images and features. In an embodiment, the ultrasound imaging device 140 may include a separate dedicated display for acquiring the ultrasound images, where dedicated display is also represented by the display 124.

Referring to the memory 130, the various modules store sets of data and instructions executable by the processor 120 to perform a sarcopenia evaluation, as mentioned above. Longitudinal scan image module 131 is configured to receive and process ultrasound images of a muscle of interest 155 in the subject 150 provided from a longitudinal scan by the ultrasound imaging device 140 (referred to as "longitudinal ultrasound images"). Transverse scan image module 132 is configured to receive and process ultrasound images of the muscle of interest 155 from a transverse scan by the ultrasound imaging device 140 (referred to as "transverse ultrasound images"). The longitudinal scan generally provides length and depth viewpoints of the muscle of interest 155 from the ultrasound transducer probe 145, and the transverse scan generally provides width and depth viewpoints of the muscle of interest 155 from the ultrasound transducer probe 145. The transverse scan may be provided at a viewpoint that is substantially perpendicular the viewpoint of the longitudinal scan, as shown by the arrows in FIG. 2. However, it is understood that the longitudinal and transverse scans may differ anywhere from one another by about 45 degrees to about 135 degrees without departing from the scope of the present teachings.

The longitudinal and transverse ultrasound images may be displayed on the display 124. The longitudinal and transverse ultrasound images may be received in real-time or near real-time from the ultrasound imaging device 140 during a contemporaneous imaging session of the subject 150. The display of real-time images, in particular, enables the user to visualize the anatomy of the subject 150 while manipulating the ultrasound transducer probe 145. Alternatively, or in addition, the longitudinal and transverse ultrasound images may be previously acquired images, obtained during previous imaging session(s), which have been retrieved from storage (e.g., database 112).

Segmentation module 133 receives a longitudinal ultrasound image from the longitudinal scan image module 131 and a transverse ultrasound image from the transverse scan image module 132 of the same muscle of interest 155, and performs segmentation of the muscle of interest 155 in each of the received longitudinal and transverse ultrasound images. The segmentation may be performed using a known conventional image segmentation method or a deep learning-based segmentation algorithm, for example, as would be apparent to one skilled in the art. In an embodiment, the deep learning-based segmentation algorithm may be a convolutional neural network (CNN), such as U-Net, for example. Alternatively, or in addition, at least a portion of the segmentation and/or edits to the automated segmentation may be performed manually by the user. In various embodiments, the segmentation of the longitudinal ultrasound image may be performed using the same or different method or algorithm than that used to perform the segmentation of the transverse ultrasound image. The segmentation provides a first targeted muscle region 151 of the muscle of interest 155 from the longitudinal ultrasound image, and a second targeted muscle region 153 of the muscle of interest 155 from the transverse ultrasound image.

In an embodiment, the deep-learning based segmentation algorithm mentioned above may be implemented as a N-fold voting mechanism, for example, that executes N differently trained prediction models, where N is a positive integer. In an embodiment, N may be less than or equal to 10, for example, since N greater than 10 may lead to undesirably long processing times. According to the N-fold voting mechanism, training sets and validation sets are equally divided into N folds. Each of the folds is then sequentially selected as validation set, while the remaining N-1 folds provide a training set, to train N prediction models using these N different data selections. All of the prediction models are used to predict the muscle segmentation and to vote for the final result, e.g., by averaging the predicted segmentations to get the final result. Since the user has the opportunity to edit boundaries of the targeted muscle regions, as discussed below, one prediction model (N = 1) is typically sufficient for performing the deep-learning based segmentation algorithm. A 10-fold voting mechanism (N = 10), for example, generally improves performance of the deep-learning based segmentation algorithm slightly, but requires significantly more time and computational power to execute.

In an embodiment, the segmentation module 133 also determines the quality of the muscle segmentations for the longitudinal and transverse ultrasound images, and whether the quality is acceptable. For example, the segmentation module 133 may determine the quality of the muscle segmentations automatically by identifying what portion of the segmented ultrasound images has regional segmentation quality that is not usable, e.g., due to contours that are not smooth and/or strange concaves. Alternatively, the segmentation module 133 may cause the segmentation results to be displayed on the display 124, enabling the user to judge the quality of the muscle segmentations. In this case, the segmentation module 133 receives a quality indication from the user via the user interface 122 once the user has viewed the muscle segmentations.

When the quality of the muscle segmentation is not acceptable, the segmentation module 133 may perform fine-tuning of the muscle segmentation. This may be done by regional re-segmentation. Alternatively, when the quality of the muscle segmentation is not acceptable, the user may manually edit the muscle segmentations using the user interface 122. For example, the user may perform fine tuning by manually adjusting the borders of the targeted muscle regions and/or the small ROIs in the longitudinal and/or transverse ultrasound images for the muscle of interest 155.

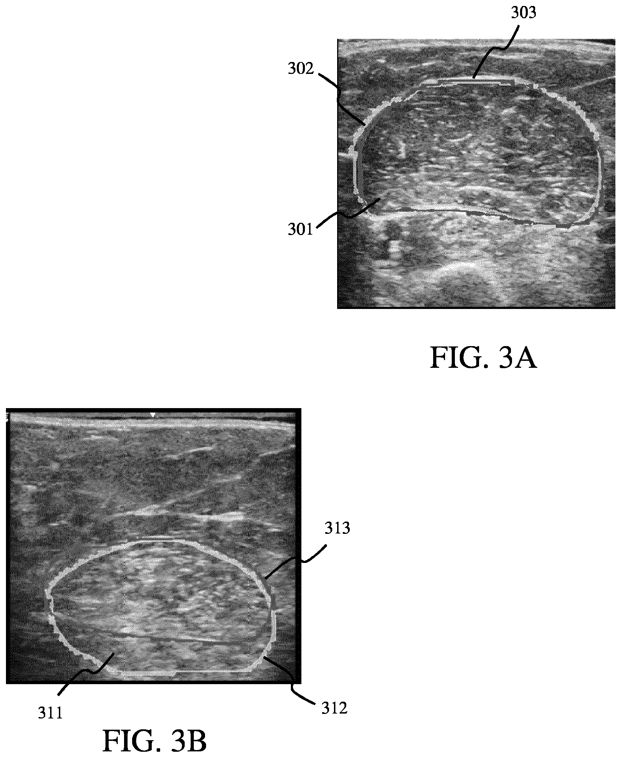

FIG. 3A shows excellent segmentation results by the deep learning-based segmentation algorithm, according to a representative embodiment. Referring to FIG. 3A, targeted muscle region 301 is shown with a ground truth border 302, and a segmented (predicted) border 303 following the muscle segmentation. Comparison of the ground truth border 302 and the segmented border 303 shows excellent alignment, where the Dice similarity coefficient is above 0.98. Thus, only minor small fine-tuning is required by the user to adjust the segmented border 303 of the targeted muscle region 301 to substantially match the ground truth border.

In comparison, FIG. 3B shows relatively moderate to poor muscle segmentation results by the deep learning-based segmentation algorithm, according to a representative embodiment. Referring to FIG. 3B, targeted muscle region 311 is shown with a ground truth border 312, and a segmented (predicted) border 313 following the muscle segmentation. Comparison of the ground truth border 312 and the segmented border 313 shows fairly significant misalignment, where the Dice similarity coefficient is about 0.72. The misalignment may be due to a limited data size for the deep-learning based segmentation algorithm, for example. Thus, editing by the user is needed to correct the mismatching boundary at the bottom of the targeted muscle region 311.

Referring again to FIG. 2, global attenuation module 134 is configured to automatically compute global attenuation (dB/cm) for each of the first and second targeted muscle regions 151 and 153 for the longitudinal scan and the transverse scan of the muscle of interest 155, respectively. The global attenuations are determined within the boundaries of the first and second targeted muscle regions 151 and 153 using any known technique. For example, a first global attenuation in the first targeted muscle region 151 from the longitudinal scan may be determined between the upper and lower boundaries, while a second global attenuation in the second targeted muscle region 153 from the transverse scan may be determined between the left and right boundaries. The boundaries of the longitudinal and transverse scans are used for averaged global attenuation for each of the entire first and second targeted muscle regions 151 and 153 in a single frame of the longitudinal and transverse ultrasound images. Generally, the value of the global attenuation decreases as the amount of fat relative to muscle increases. In other words, the denser the tissue (i.e., the greater the proportion of muscle), the greater the global attenuation of ultrasound waves through the tissue.

Local attenuation module 135 is configured to automatically compute local attenuation coefficients (dB/cm/MHz) for small first and second regions of interest (ROIs) 152 an 154 within the first and second targeted muscle regions 151 and 153 for the longitudinal scan and the transverse scan, respectively, using any known technique. The local attenuation module 135 enables selection of the first ROI 152 within the first targeted muscle region 151 from a single image frame, and selection of the second ROI 154 within the second targeted muscle region 153 from a single image frame. The selection of the first and second ROIs 152 and 154 may be done automatically by segmentation, or manually by the user through the user interface 122. For example, each of the first and second ROIs 152 and 154 may be selected by the user via the user interface 122 or may be set automatically to a specific ROI at the middle region of the targeted muscle regions 151 and 153, respectively. For example, the middle regions of the targeted muscle regions 151 and 153 may be identified and cropped automatically from the respective muscle segmentations to provide the first and second ROIs 152 and 154. Once the first and second ROIs 152 and 154 have been identified, a first local attenuation coefficient may be determined for the first ROI 152 from the longitudinal scan, and a second local attenuation coefficient may be determined for the second ROI 154 from the transverse scan. The local attenuation coefficients may be calculated for the first and second ROIs 152 and 154 directly or by averaging the corresponding attenuation maps.

A method for determining a local attenuation coefficient for fatty liver quantification, for example, may be adapted to determine the local attenuation coefficient for targeted muscle regions, as well. For example, a linear ultrasound probe may be used for imaging the targeted muscle region instead of a curved ultrasound probe used for imaging the liver. Generally, the average of a local attenuation coefficient of a normal liver is about 0.567 dB/cm/MHz, the average of a local attenuation coefficient of a mild fatty liver is about 0.659 dB/cm/MHz, and the average of a local attenuation coefficient of a severe fatty liver is about 0.789 dB/cm/MHz. The relative values among averages of normal, mild fatty and severe fatty targeted muscle regions will be similar.

Global SoS module 136 is configured to automatically compute global SoS (m/s) of the ultrasound waves in each of the first and second targeted muscle regions 151 and 153 for the longitudinal scan and the transverse scan of the muscle of interest 155, respectively. The global SoS values are determined within the boundaries of the first and second targeted muscle regions 151 and 153, respectively, by calculating the propagation speed of sound waves through the tissues, for example, using any known technique. For example, a first global SoS in the first targeted muscle region 151 from the longitudinal scan may be determined by averaging SoS values through the upper and lower boundaries, while a second global SoS in the second targeted muscle region 153 region from the transverse scan may be determined by averaging SoS values through the left and right boundaries. Generally, the value of the global SoS decreases as the amount of fat relative to muscle increases. In other words, the denser the tissue (i.e., the greater the proportion of muscle to fat), the higher the speed of ultrasound waves through the tissue, since the SoS of ultrasound waves through fat tissue is about 1440 m/s, while the SoS of ultrasound waves through muscle tissue is about 1585 m/s.

Local SoS module 137 is configured to automatically compute local SoS (m/s) of the ultrasound waves in each of the first and second ROIs 152 and 154 within the first and second targeted muscle regions 151 and 153 for the longitudinal scan and the transverse scan of the muscle of interest, respectively, using any known technique. For example, a first local SoS may be determined for the first ROI 152 and a second local SoS may be determined for the second ROI 154. Each of the first and second ROIs 152 and 154 may be selected by the user or may be set to a specific ROI at the middle region of the first and second targeted muscle regions 151 and 153, respectively. The local SoSs may be calculated for the first and second ROIs 152 and 154 directly or by averaging the respective attenuation maps, for example.

Sarcopenia grading module 138 is configured to determine the level (grade) of sarcopenia in the muscle of interest 155 (e.g., normal sarcopenia, mild sarcopenia, moderate sarcopenia, severe sarcopenia), thereby grading the sarcopenia. For example, normal sarcopenia (i.e., little to no sarcopenia) may receive a grade of 0 or A, mild sarcopenia may receive a grade of 1 or B, moderate sarcopenia may receive a grade of 2 or C, and severe sarcopenia made receive a grade of 3 or D. The different levels are defined by corresponding predetermined thresholds. The level of sarcopenia may be calculated by applying a regression model to a combination of the eight parameters output by the global attenuation module 134, the local attenuation module 135, global SoS module 136, and the local SoS module 137, where four of the parameters are from the longitudinal ultrasound image and four of the parameters are from the transverse ultrasound image. The regression model will determine a weight for each of the eight parameters, and combine with the weighted parameters to output the level of sarcopenia based on the predetermined thresholds.

In various embodiments, the regression model may be a machine-learning algorithm, such as neural network model, for example, such as an artificial a neural network (ANN), a recurrent neural network (RNN), or a CNN. The regression model therefore may be trained using previously acquired data that provides values of the eight parameters and corresponding levels of sarcopenia in previous patients. The training data may be stored in the database 112, for example. The training also compares the relative effects of the eight parameters on the resulting levels of sarcopenia in order to establish the degrees to which the eight parameters influence the final level of sarcopenia. Based on these relative effects, the regression model determines and assigns the respective weights to the eight parameters. Current sarcopenia results and corresponding parameters may be added to this training database to continue to hone accuracy of the regression model. That is, the training minimizes similarity loss in order to improve accuracy of the regression model as the training dataset grows, as would be apparent to one skilled in the art.

The weights applied by the regression model may account for various different relationships among the parameters, depending on the corresponding influences on the overall determination of sarcopenia. For example, the global parameters (i.e., global attenuations and global SoSs) from each of the longitudinal and transverse ultrasound images may be weighted heavier than the local parameters (i.e., local attenuation coefficients and local SoSs) from each of the longitudinal and transverse ultrasound images. Also, the measures of attenuations (i.e., global attenuations and local attenuation coefficients) in each of the longitudinal and transverse ultrasound images may be weighted slightly heavier than the measures of SoS (i.e., global and local SoSs) in each of the longitudinal and transverse ultrasound images.

In various embodiments, all or part of the processes provided by the machine-learning regression model may be implemented by an AI engine, for example, mentioned above. The neural network model may be trained using ground truth longitudinal and transverse ultrasound images of targeted muscle regions and ROIs in a muscle of interest in multiple patients. The longitudinal and transverse ultrasound images are associated with corresponding global attenuations, local attenuation coefficients, global SoSs, and local SoSs determined for each, as well as resulting sarcopenia grades. Therefore, during the training phase, the neural network model will learn appropriate sarcopenia levels from retrospective data t be applied to current parameters.

An indication of the resulting sarcopenia level is provided to the user, e.g., via the display 124, diagnosing whether sarcopenia exists in the subject 150 and, if so, the level of the sarcopenia (e.g., mild to severe) in the subject. 150. In an embodiment, the sarcopenia level may be provided to a reporting module (not shown), which formats the information from the sarcopenia grading module 138, and causes the formatted information to be displayed on the display 124 via the GUI 128. In addition to the sarcopenia level, the displayed information may include all or some of the parameters used by the sarcopenia grading module 138 to determine the level of sarcopenia, including the global attenuations, the local attenuation coefficients, the global SoSs, and the local SoSs from the longitudinal and transverse ultrasound images. Also, in an embodiment, the longitudinal and transverse images themselves may be displayed along with the sarcopenia grade, and any additional information. Appropriate medical treatment may then be provided in view of the diagnosis.

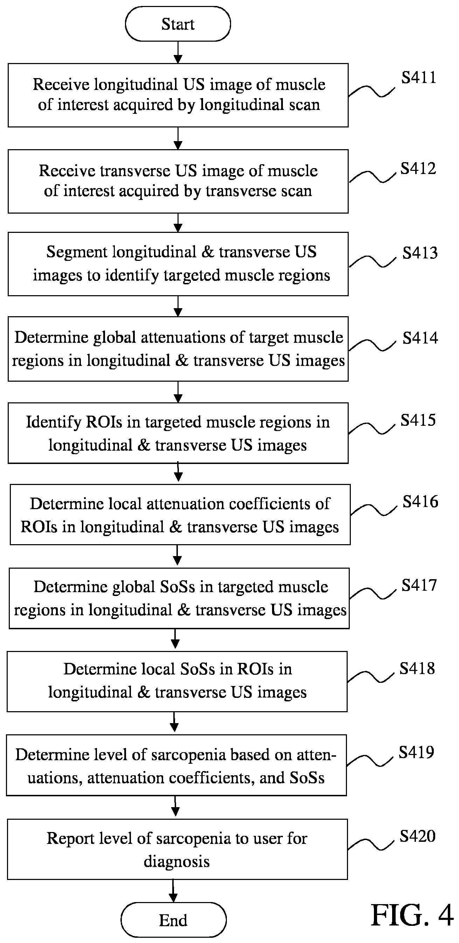

FIG. 4 is a flow diagram of a method of evaluating sarcopenia in a subject using ultrasound imaging. The method may be implemented using instructions stored in memory 130 and executable by the processor 120 in the system 100, for example. In an embodiment, the method may be implemented by an on-line version for improving MSK ultrasound workflow of an ultrasound imaging device, e.g., for muscle function evaluation targeting of subjects with high-risk of developing severe sarcopenia.

Referring to FIG. 4, a longitudinal ultrasound image is received in block S411 by a processer (e.g., processor 120), where the longitudinal ultrasound image has been acquired by a longitudinal scan of a muscle of interest in the patient using ultrasound imaging. The longitudinal scan has been performed after pre-setting the ultrasound imaging device for MSK imaging. The longitudinal ultrasound image may be received in real-time or near real-time from an ultrasound imaging device (e.g., ultrasound imaging device 140), during an ultrasound imaging session, or may be retrieved from a database (e.g., database 112) storing images acquired during a previous ultrasound imaging session.

In block S412, a transverse ultrasound image is received by the processer, where the transverse ultrasound image has been acquired by a transverse scan of the muscle of interest in the patient using ultrasound imaging. The transverse ultrasound image may be received in real-time or near real-time from the ultrasound imaging device during the ultrasound imaging session, or may be retrieved from the database.

In block S413, the longitudinal and transverse ultrasound images are segmented to identify targeted muscle regions of the muscle of interest, respectively. That is, the longitudinal ultrasound image is segmented to identify a first targeted muscle region and the transverse ultrasound image is segmented to identify a second targeted muscle region.

In block S414, global attenuations in the target muscle regions are determined between boundaries of the target muscle regions in the longitudinal and transverse ultrasound images, respectively. That is, a first global attenuation in the first targeted muscle region is determined between upper and lower boundaries defining the first targeted muscle region and a second global attenuation in the second targeted muscle region is determined between left and right boundaries defining the second targeted muscle region.

In block S415, regions of interest (ROIs) are identified in the targeted muscle regions in the longitudinal and the transverse ultrasound images, respectively. That is, a first ROI is identified in the first targeted muscle region and a second ROI is identified in the second targeted muscle region. Each of the first ROI and the second ROI may be identified manually by the user, or may be identified automatically by the processor. For example, the first ROI may be identified at the middle region of the first targeted muscle region and the second ROI may be identified at the middle region of the second targeted muscle region, where the middle regions may be automatically identified and cropped from the segmented target muscle regions.

In block S416, local attenuation coefficients of the ROIs are determined in the targeted muscle regions, respectively. That is, a first local attenuation coefficient is determined for the first ROI in the first targeted muscle region and a second local attenuation coefficient is determined for the second ROI in the second targeted muscle region.

In block S417, global SoSs in the target muscle regions are determined between boundaries of the targeted muscle regions in the longitudinal and transverse ultrasound images, respectively. That is, a first global SoS in the first targeted muscle region is determined between upper and lower boundaries defining the first targeted muscle region and a second global SoS in the second targeted muscle region is determined between left and right boundaries defining the second targeted muscle region.

In block S418, local SoSs in the ROIs in the targeted muscle regions are determined in the longitudinal and transverse ultrasound images, respectively. That is, a first local SoS in the first ROI is determined in the first targeted muscle region and a second local SoS in the second ROI is determined in the second targeted muscle region.

In block S419, a level of sarcopenia in the subject is determined (graded) based on the global attenuations and the global SoSs in the first and second targeted muscle regions, and the local attenuation coefficients and the local SOSs through the first and second ROIs. The level of sarcopenia may be determined by applying a regression model to the global attenuations and the global SoSs of the first and second targeted muscle regions and the local attenuation coefficients and the local SoSs of the first and second ROIs, respectively, as discussed above.

In block S420, the level of sarcopenia is reported to the user via a display or GUI as a diagnostic result for the patient. The user is able to determine an appropriate course of medical treatment, if any, accordingly. The reporting may further include displaying a representative image frame for each of the longitudinal scan and the transverse scan. Also, one or more of the first global attenuation, local attenuation coefficient, global SoS, and local SoS may be displayed with the image frame for the longitudinal scan, and one or more of the second global attenuation, local attenuation coefficient, global SoS, and local SoS may be displayed with the image frame for the transverse scan.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although evaluating sarcopenia using ultrasound imaging has been described with reference to exemplary embodiments, it is understood that the words that have been used are words of description and illustration, rather than words of limitation. Changes may be made within the purview of the appended claims, as presently stated and as amended, without departing from the scope of the embodiments. Although evaluating sarcopenia using ultrasound imaging has been described with reference to particular means, materials and embodiments, it is not intended to be limited to the particulars disclosed; rather evaluating sarcopenia using ultrasound imaging extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A system for diagnosing sarcopenia in a patient (150), the system comprising:
a user interface (122);
a processor (120) in communication with the user interface;
a display configured to display an indication of the level of sarcopenia,
system **characterized in that** it further comprises a non-transitory memory (130) storing instructions that, when executed by the processor, cause the processor to:
receive a longitudinal ultrasound image acquired by a longitudinal scan of a muscle of interest (155) in the patient using ultrasound imaging (S411);
receive a transverse ultrasound image acquired by a transverse scan of the muscle of interest in the patient using ultrasound imaging (S412);
segment the longitudinal and transverse ultrasound images to identify targeted muscle regions (151, 153) of the muscle of interest in the longitudinal and the transverse ultrasound images, respectively (S413);
determine global attenuations in the target muscle regions between boundaries in the longitudinal and transverse ultrasound images, respectively (S414);
identify regions of interest (ROIs) (152, 154) in the targeted muscle regions in the longitudinal and the transverse ultrasound images, respectively (S415);
determine local attenuation coefficients of the ROIs in the targeted muscle regions, respectively (S416);
determine global speeds of sound (SoSs) in the targeted muscle regions between the boundaries in the longitudinal and transverse ultrasound images, respectively (S417);
determine local SoSs in the ROIs in the targeted muscle regions, respectively (S418); and
determine a level of sarcopenia in the patient based on the global attenuations and the global SoSs in the targeted muscle regions, and the local attenuation coefficients and the local SOSs in the ROIs (S419).

2. The system of claim 1, wherein the instructions cause the processor to segment the longitudinal and transverse ultrasound images by applying a deep learning-based segmentation algorithm to image data from the longitudinal and transverse images.

3. The system of claim 2, wherein segmenting the longitudinal and transverse ultrasound images further comprises fine-tuning or editing at least one mismatching boundary of at least one of the targeted muscle regions.

4. The system of claim 1, wherein the instructions cause the processor to determine the level of sarcopenia in the patient by applying a regression model to the global attenuations and the global SoSs in the targeted muscle regions, and the local attenuation coefficients and the local SOSs in the ROIs.

5. The system of claim 1, wherein the instructions cause the processor to identify the ROIs of the targeted muscle regions by:
receiving a selection of the local attenuation coefficients of the ROIs from a user at the user interface; or
selecting automatically the ROIs at a middle region of the targeted muscle regions.

6. The system of claim 5, wherein the instructions cause the processor to determine the local attenuation coefficients of the ROIs by averaging attenuation maps from the ROIs, respectively.

7. The system of claim 1, wherein the instructions cause the processor to determine the global SoSs through the targeted muscle regions by averaging the SoSs through the boundaries of the targeted muscle regions, respectively.

8. The system of claim 7, wherein determining the local SoSs through the ROIs comprises averaging SoS maps from the ROIs, respectively.

9. The system of claim 1, wherein the display is further configured to:
display a representative image frame for each of the longitudinal scan and the transverse scan;
display one or more of the global attenuation, the local attenuation coefficient, the global SoS, and the local SoS associated with the longitudinal ultrasound image with the representative image frame for the longitudinal scan; and
display one or more of the global attenuation, the local attenuation coefficient, the global SoS, and the local SoS associated with the transverse ultrasound image with the representative image frame for the transverse scan.

10. A non-transitory computer readable medium (130) storing instructions for diagnosing sarcopenia in a patient (150) that, when executed by a processor, cause the processor to:
receive a longitudinal ultrasound image acquired by a longitudinal scan of a muscle of interest (155) in the patient using ultrasound imaging (S411);
receive a transverse ultrasound image acquired by a transverse scan of the muscle of interest in the patient using ultrasound imaging (S412);
segment the longitudinal and transverse ultrasound images to identify targeted muscle regions (151, 153) of the muscle of interest in the longitudinal and the transverse ultrasound images, respectively (S413);
determine global attenuations in the target muscle regions between boundaries in the longitudinal and transverse ultrasound images, respectively (S414);
identify regions of interest (ROIs) (152, 154) in the targeted muscle regions in the longitudinal and the transverse ultrasound images, respectively (S415);
determine local attenuation coefficients of the ROIs in the targeted muscle regions, respectively (S416);
determine global speeds of sound (SoSs) in the targeted muscle regions between the boundaries in the longitudinal and transverse ultrasound images, respectively (S417);
determine local SoSs in the ROIs in the targeted muscle regions, respectively (S418);
determine a level of sarcopenia in the patient based on the global attenuations and the global SoSs in the targeted muscle regions, and the local attenuation coefficients and the local SOSs in the ROIs (S419); and
display an indication of the level of sarcopenia (S420).

## Patentansprüche

1. System zum Diagnostizieren von Sarkopenie bei einem Patienten (150), wobei das System umfasst:
eine Benutzerschnittstelle (122);
einen Prozessor (120), der mit der Benutzerschnittstelle in Kommunikation steht;
eine Anzeige, die so konfiguriert ist, dass sie eine Angabe des Sarkopeniegrades anzeigt,
wobei das System **dadurch gekennzeichnet ist, dass** es weiter einen nichtflüchtigen Speicher (130) umfasst, der Anweisungen speichert, die, wenn sie von dem Prozessor ausgeführt werden, bewirken, dass der Prozessor:
ein Längsultraschallbild empfängt, das durch einen Längsscan eines Muskels von Interesse (155) bei dem Patienten unter Verwendung von Ultraschallbildgebung erfasst wird (S411);
ein Querultraschallbild empfängt, das durch einen Querscan des Muskels von Interesse bei dem Patienten unter Verwendung von Ultraschallbildgebung erfasst wird (S412);
jeweils das Längs- und Querultraschallbild segmentiert, um Zielmuskelregionen (151, 153) des Muskels von Interesse in dem Längs- und dem Querultraschallbild zu identifizieren (S413);
jeweils globale Dämpfungen in den Zielmuskelregionen zwischen Begrenzungen in dem Längs- und dem Querultraschallbild bestimmt (S414);
jeweils Regionen von Interesse (ROls) (152, 154) in den Zielmuskelregionen in dem Längs- und dem Querultraschallbild identifiziert (S415);
jeweils lokale Dämpfungskoeffizienten der ROIs in den Zielmuskelregionen bestimmt (S416);
jeweils globale Schallgeschwindigkeiten (SoSs) in den Zielmuskelregionen zwischen den Begrenzungen in dem Längs- und Querultraschallbild bestimmt (S417);
jeweils lokale SoSs in den ROs in den Zielmuskelregionen bestimmt (S418); und
einen Sarkopeniegrad bei dem Patienten auf Basis der globalen Dämpfungen und der globalen SoSs in den Zielmuskelregionen, und der lokalen Dämpfungskoeffizienten und der lokalen SoSs in den ROIs bestimmt (S419).

2. System nach Anspruch 1, wobei die Anweisungen bewirken, dass der Prozessor das Längs- und Querultraschallbild durch Anwenden eines auf Deep Learning basierenden Segmentierungsalgorithmus auf Bilddaten aus dem Längs- und Querbild segmentiert.

3. System nach Anspruch 2, wobei das Segmentieren des Längs- und Querultraschallbildes weiter das Feinabstimmen oder Bearbeiten mindestens einer nicht übereinstimmenden Begrenzung mindestens einer der Zielmuskelregionen umfasst.

4. System nach Anspruch 1, wobei die Anweisungen bewirken, dass der Prozessor den Sarkopeniegrad bei dem Patienten durch Anwenden eines Regressionsmodells auf die globalen Dämpfungen und die globalen SoSs in den Zielmuskelregionen, und die lokalen Dämpfungskoeffizienten und die lokalen SoSs in den ROIs bestimmt.

5. System nach Anspruch 1, wobei die Anweisungen bewirken, dass der Prozessor die ROIs der Zielmuskelregionen identifiziert durch:
Empfangen einer Auswahl der lokalen Dämpfungskoeffizienten der ROIs von einem Benutzer an der Benutzerschnittstelle; oder
automatisches Auswählen der ROIs in einer mittleren Region der Zielmuskelregionen.

6. System nach Anspruch 5, wobei die Anweisungen bewirken, dass der Prozessor die lokalen Dämpfungskoeffizienten der ROIs jeweils durch Mitteln von Dämpfungskarten aus den ROIs bestimmt.

7. System nach Anspruch 1, wobei die Anweisungen bewirken, dass der Prozessor die globalen SoSs durch die Zielmuskelregionen jeweils durch Mitteln der SoSs durch die Begrenzungen der Zielmuskelregionen bestimmt.

8. System nach Anspruch 7, wobei das Bestimmen der lokalen SoSs durch die ROIs das jeweilige Mitteln von SoS-Karten aus den ROIs umfasst.

9. System nach Anspruch 1, wobei die Anzeige weiter so konfiguriert ist, dass sie:
für jeden des Längsscans und des Querscans ein repräsentatives Einzelbild anzeigt;
eines oder mehrere der globalen Dämpfung, des lokalen Dämpfungskoeffizienten, der globalen SoS und der lokalen SoS, die dem Längsultraschallbild zugeordnet sind, zusammen mit dem repräsentativen Einzelbild für den Längsscan anzeigt; und
eines oder mehrere der globalen Dämpfung, des lokalen Dämpfungskoeffizienten, der globalen SoS und der lokalen SoS, die dem Querultraschallbild zugeordnet sind, zusammen mit dem repräsentativen Einzelbild für den Querscan anzeigt.

10. Nichtflüchtiges computerlesbares Medium (130), das Anweisungen zum Diagnostizieren von Sarkopenie bei einem Patienten (150) speichert, die, wenn sie von einem Prozessor ausgeführt werden, bewirken, dass der Prozessor:
ein Längsultraschallbild empfängt, das durch einen Längsscan eines Muskels von Interesse (155) bei dem Patienten unter Verwendung von Ultraschallbildgebung erfasst wird (S411);
ein Querultraschallbild empfängt, das durch einen Querscan des Muskels von Interesse bei dem Patienten unter Verwendung von Ultraschallbildgebung erfasst wird (S412);
jeweils das Längs- und Querultraschallbild segmentiert, um Zielmuskelregionen (151, 153) des Muskels von Interesse in dem Längs- und dem Querultraschallbild zu identifizieren (S413);
jeweils globale Dämpfungen in den Zielmuskelregionen zwischen Begrenzungen in dem Längs- und dem Querultraschallbild bestimmt (S414);
jeweils Regionen von Interesse (ROls) (152, 154) in den Zielmuskelregionen in dem Längs- und dem Querultraschallbild identifiziert (S415);
jeweils lokale Dämpfungskoeffizienten der ROIs in den Zielmuskelregionen bestimmt (S416);
jeweils globale Schallgeschwindigkeiten (SoSs) in den Zielmuskelregionen zwischen den Begrenzungen in dem Längs- und Querultraschallbild bestimmt (S417);
jeweils lokale SoSs in den ROIs in den Zielmuskelregionen bestimmt (S418);
einen Sarkopeniegrad bei dem Patienten auf Basis der globalen Dämpfungen und der globalen SoSs in den Zielmuskelregionen, und der lokalen Dämpfungskoeffizienten und der lokalen SoSs in den ROIs bestimmt (S419); und
eine Angabe des Sarkopeniegrades anzeigt (S420).

## Revendications

1. Système de diagnostic de la sarcopénie chez un patient (150), le système comprenant :
une interface utilisateur (122) ;
un processeur (120) en communication avec l'interface utilisateur ;
un écran configuré pour afficher une indication du niveau de sarcopénie,
le système étant **caractérisé en ce qu'**il comprend en outre une mémoire non transitoire (130) stockant des instructions qui, lorsqu'elles sont exécutées par le processeur, amènent le processeur à :
recevoir une image ultrasonore longitudinale acquise par un balayage longitudinal d'un muscle d'intérêt (155) chez le patient à l'aide de l'imagerie ultrasonore (S411) ;
recevoir une image échographique transversale acquise par un balayage transversal du muscle d'intérêt chez le patient à l'aide de l'imagerie échographique (S412) ;
segmenter les images ultrasonores longitudinales et transversales pour identifier les régions musculaires ciblées (151, 153) du muscle d'intérêt dans les images ultrasonores longitudinales et transversales, respectivement (5413) ;
déterminer les atténuations globales dans les régions musculaires cibles entre les limites dans les images ultrasonores longitudinales et transversales, respectivement (5414) ;
identifier les régions d'intérêt (ROI) (152, 154) dans les régions musculaires ciblées dans les images ultrasonores longitudinales et transversales, respectivement (S415) ;
déterminer les coefficients d'atténuation locaux des ROI dans les régions musculaires ciblées, respectivement (5416) ;
déterminer les vitesses du son (SoS) globales dans les régions musculaires ciblées entre les limites des images ultrasonores longitudinales et transversales, respectivement (S417) ;
déterminer les SoS locales dans les ROI des régions musculaires ciblées, respectivement (5418) ; et
déterminer un niveau de sarcopénie chez le patient sur la base des atténuations globales et des SoS globales dans les régions musculaires ciblées, et des coefficients d'atténuation locaux et des SOS locales dans les ROI (S419).

2. Système selon la revendication 1, dans lequel les instructions amènent le processeur à segmenter les images ultrasonores longitudinales et transversales en appliquant un algorithme de segmentation basé sur l'apprentissage profond aux données d'image provenant des images longitudinales et transversales.

3. Système selon la revendication 2, dans lequel la segmentation des images ultrasonores longitudinales et transversales comprend en outre l'ajustement ou la modification d'au moins une limite non concordante d'au moins une des régions musculaires ciblées.

4. Système selon la revendication 1, dans lequel les instructions amènent le processeur à déterminer le niveau de sarcopénie chez le patient en appliquant un modèle de régression aux atténuations globales et aux SoS globales dans les régions musculaires ciblées, ainsi qu'aux coefficients d'atténuation locaux et aux SOS locales dans les ROI.

5. Système selon la revendication 1, dans lequel les instructions amènent le processeur à identifier les ROI des régions musculaires ciblées par :
la réception d'une sélection des coefficients d'atténuation locaux des ROI de la part d'un utilisateur au niveau de l'interface utilisateur ; ou
la sélection automatique des ROI dans une région médiane des zones musculaires ciblées.

6. Système selon la revendication 5, dans lequel les instructions amènent le processeur à déterminer les coefficients d'atténuation locaux des ROI en faisant la moyenne des cartes d'atténuation des ROI, respectivement.

7. Système selon la revendication 1, dans lequel les instructions amènent le processeur à déterminer les SoS globales à travers les régions musculaires ciblées en faisant la moyenne des SoS à travers les limites des régions musculaires ciblées, respectivement.

8. Système selon la revendication 7, dans lequel la détermination des SoS locales à travers les ROI comprend la moyenne des cartes SoS des ROI, respectivement.

9. Système selon la revendication 1, dans lequel l'écran est en outre configuré pour :
afficher une trame d'image représentative pour chacun du balayage longitudinal et du balayage transversal ;
afficher un ou plusieurs parmi l'atténuation globale, le coefficient d'atténuation locale, la SoS globale et la SoS locale associés à l'image ultrasonore longitudinale avec la trame d'image représentative pour le balayage longitudinal ; et
afficher un ou plusieurs parmi l'atténuation globale, le coefficient d'atténuation locale, la SoS globale et la SoS locale associés à l'image ultrasonore transversale avec la trame d'image représentative pour le balayage transversal.

10. Support non transitoire lisible par ordinateur (130) stockant des instructions pour le diagnostic de la sarcopénie chez un patient (150) qui, lorsqu'elles sont exécutées par un processeur, amènent le processeur à :
recevoir une image ultrasonore longitudinale acquise par un balayage longitudinal d'un muscle d'intérêt (155) chez le patient à l'aide de l'imagerie ultrasonore (S411) ;
recevoir une image échographique transversale acquise par un balayage transversal du muscle d'intérêt chez le patient à l'aide de l'imagerie échographique (S412) ;
segmenter les images ultrasonores longitudinales et transversales pour identifier les régions musculaires ciblées (151, 153) du muscle d'intérêt dans les images ultrasonores longitudinales et transversales, respectivement (5413) ;
déterminer les atténuations globales dans les régions musculaires cibles entre les limites dans les images ultrasonores longitudinales et transversales, respectivement (5414) ;
identifier les régions d'intérêt (ROI) (152, 154) dans les régions musculaires ciblées dans les images ultrasonores longitudinales et transversales, respectivement (S415) ;
déterminer les coefficients d'atténuation locaux des ROI dans les régions musculaires ciblées, respectivement (5416) ;
déterminer les vitesses du son (SoS) globales dans les régions musculaires ciblées entre les limites des images ultrasonores longitudinales et transversales, respectivement (S417) ;
déterminer les SoS locales dans les ROI des régions musculaires ciblées, respectivement (5418) ;
déterminer un niveau de sarcopénie chez le patient sur la base des atténuations globales et des SoS globales dans les régions musculaires ciblées, et des coefficients d'atténuation locaux et des SOS locales dans les ROI (S419) ; et
afficher une indication du niveau de sarcopénie (S420).
